# EUROPEAN PATENT SPECIFICATION

(11) **EP 0 686 039 B1**
(45) Date of publication and mention of the grant of the patent: **23.08.2000**
(21) Application number: 94909696.0
(22) Date of filing: 23.02.1994
(51) Int. Cl.: A61K 38/00, C12N 1/12, C12N 1/20

(54) **METHOD OF ENHANCING IMMUNE RESPONSE TO ORAL VACCINES**
METHODE ZUR VERSTÄRKUNG DER IMMUNANTWORT AUF ORALE VAKZINE
PROCEDE D'AMELIORATION DE LA REPONSE IMMUNITAIRE AUX VACCINS ORAUX

(30) Priority: 24.02.1993 US 21798
(43) Date of publication of application: 13.12.1995
(73) Proprietor: Valio Ltd., 00039 Valio (FI); Gorbach, Sherwood L., Weston, MA 02493 (US)
(72) Inventor: GORBACH, Sherwood L., Weston, MA 02493 (US); ISOLAURI, Erika, FIN-00101 Helsinki (FI); SALMINEN, Seppo J., FIN-00270 Helsinki (FI)
(74) Representative: Boije-Backman, Solveig Magdalena
(86) International application number: US9401764
(87) International publication number: WO9418997

(56) References cited:
- US-A- 4 238 478
- US-A- 4 397 838
- US-A- 4 839 281
- US-A- 5 032 399
- US-A- 5 185 321
- DATABASE MEDLINE FILE SERVER STN KARLSRUHE ABSTRACT 94074844, ISOLAURI ET AL: "LACTOBACILLUS CASEI STRAIN GG REVERSES INCREASED INTESTINAL PERMEABILITY INDUCED BY COW MILK IN SUCKLING RATS" XP002029965
- Pediatric Research, Volume 32, No. 2, issued 1992, M. KAILA et al.: "Enhancement of the Circulating Antibody Secreting Cell Response in Human Diarrhea by a Human Lactobacillus Strain", pages 141-144, entire document.
- Journal of Food Protection, Volume 53, No. 5, issued May 1990, G. PERDIGON et al.: "The Oral Administration of Lactic Acid Bacteria Increase the Mucosal Intestinal Immunity in Response to Enteropathogens", pages 404-410, entire document.
- Biken Journal, Volume 18, No. 2, issued June 1975, S. KOTANI et al.: "Immunoadjuvant Activities of Peptidoglycan Subunits from the Cell Walls of Staphylococcus Aureus and Lactobacillus Plantarum", pages 93-103.
- Res. Microbiol., Volume 141, issued 1990, K. GERRITSE et al.: "Oral Administration of TNP-Lactobacillus Conjugates in Mice: A Model for Evaluation of Mucosal and Systemic Immune Responses and Memory Formation Elicited by Transformed Lactobacilli", pages 955-962.
- Agric. Biol. Chem., Volume 48, No. 7, issued 1984, S. KAWATA et al.: "Preparation of Disaccharide Peptides with Immunostimulation from Microbial Cell Walls", pages 1783-1793.
- Journal of Dairy Research, Volume 58, No. 4, issued 1991, G. PERDIGON et al.: "Immunoadjuvant Activity of Oral Lactobacillus Casei: Influence of Dose on the Secretory Immune Response and Protective Capacity in Intestinal Infections", pages 485-496, especially pages 486 and 495.

## Description

The present invention relates to methods of enhancing immune response to oral vaccines.

The development of oral vaccines offers great promise in the prevention of disease world-wide. Vaccines which successfully immunize after a single dose are preferred as they eliminate the problems associated with making sure that each subject receives the proper number of subsequent doses.

However, some oral vaccines have inadequate immuno-genicity to ensure immunization with a single dose, particularly when administered to children.

A number of chemical substances have been used as immunoadjuvants, i.e., substances that are administered with a vaccine to enhance cell-mediated immunity or to increase the humoral response to the immunogen used for vaccination. These substances have traditionally not been used with oral vaccines because they may cause undesirable side effects or are not suitable for oral administration.

The term "immunoadjuvant", as used herein, refers to substances which enhance the immune response to a vaccine.

The present invention is based on the use of appropriate Lactobacillus bacteria as immunoadjuvants for oral vaccines. Kaila et al. (1992) Pediatric Research, 32:141-144 found that Lactobacillus GG shortened the clinical course of rotavirus diarrhea and potentiated non-specific and rotavirus antigen-specific immune responses following acute rotavirus gastroenteritis. Perdigon et al., (1990) Journal of Food Protection 53:404-410 and Perdigon et al., (1991) Journal of Dairy Research 58:485-496 disclosed the preventing effect of lactic acid bacteria on enteric infections, when the bacteria were fed prior to the challenge with enteropathogens. However, none of the cited documents refers to the use of lactic acid bacteria as immunoadjuvants for oral vaccines.

The invention features the use of an immunoadjuvant for enhancing the immune response to an oral vaccine of a recipient of the vaccine. To enhance the immune response, an immunoadjuvant comprising an effective amount of a strain of *Lactobacillus* bacteria is administered to the recipient, with the oral vaccine or close enough in time to bring about immune response enhancement.

Preferably, the *Lactobacillus* bacteria are able to survive at low pH and produce large amounts of an antimicrobial substance. Preferred strains having these properties are described in U.S. 4,839,281 and 5,032,399, the disclosures of which are incorporated herein by reference. Preferred bacteria are able to colonize the intestinal tract, as evidenced by the presence of the bacteria in the feces of the subject after oral administration, and preferably also have substantially the same properties exhibited by the Lactobacillus bacteria deposited in the American Type Culture Collection and given ATCC Accession No. 53103. Most preferably, the *Lactobacillus* bacteria is *Lactobacillus* Strain GG.

In other preferred embodiments, the immunoadjuvant is administered in a pharmaceutically acceptable carrier, e.g., a fermented dairy product or a culture-containing powder, capsule or tablet. For optimal enhancement of the immune response, the *Lactobacillus* bacteria is preferably coadministered to the person given the vaccine, then administered alone to the person for a period of one or more days after administration of the vaccine.

Advantageously, the immunoadjuvant of the invention enhances immunity after administration of an oral vaccine. The immunoadjuvant may allow immunity to be obtained from a single dose of an oral vaccine which, due to its characteristics or other factors, would otherwise require multiple doses, or may enhance the immune response to an oral vaccine. Also, as the *Lactobacillus* bacteria is a harmless naturally occurring substance, present in, e.g., yogurt, it produces no significant harmful side effects.

Other features and advantages of the invention will be apparent from the description of the preferred embodiment thereof, and from the claims.

The term "*Lactobacillus* Strain GG" as used herein refers to a particular strain of *Lactobacillus* bacteria, a lactic acid bacteria. This strain is described in U.S. Patent Nos. 4,839,281 and 5,032,399, incorporated above by reference. The GG strain has been deposited in the American Type Culture Collection (ATCC), Rockville, Mass., and has been given ATCC Accession No. 53103. *Lactobacillus* Strain GG may be cultured on any appropriate sterilized fermentation medium. When the *Lactobacillus* Strain GG is to be freeze-dried prior to addition to the vaccine, it is preferred that a cryoprotectant, e.g. monosodium glutamate sodium ascorbate, saccharose or lactose, be added to the cells after culturing.

The *Lactobacillus* Strain GG bacteria is administered in an amount which is sufficient to colonize the intestinal tract of the patient. A dosage of at least 10⁸ cfu, preferably 10⁸ to 10¹⁰ cfu, is generally adequate for most patients. Dosages of 10¹⁰ cfu are likely to completely colonize the intestinal tract of substantially all patients. Higher dosages may be administered, if desired, but dosages above about 5x10¹⁰ cfu typically require impractically large volumes of the bacteria-containing material to be administered.

It is preferred that the bacteria be administered to the patient over a series of days, with the preferred dosage (above) given on each day. Preferably, the bacteria is first coadministered with the vaccine, then administered alone for a period of 1 to 4 days, preferably 2 days, after administration of the vaccine. It has been found that this course of treatment provides optimal enhancement of the immune response.

The bacteria may be mixed with a pharmaceutically acceptable carrier or a food product, for ease of administration. For infants and small children, it is generally preferable to provide the bacteria in the form of a powder. For other patients, the bacteria may be provided in tablet or capsule form, as a freeze-dried powder which may be mixed with food or drink, or in any other suitable form. The bacteria may also be provided in the form of a *Lactobacillus*-containing cultured dairy product, provided the product contains an effective amount of the bacteria.

Oral vaccines which are suitable for use in the invention include, but are not limited to, vaccines for rotavirus, typhoid, cholera, adenovirus, polio, E. coli and the like.

Preferred embodiments of the invention have been described herein. Other variations and modifications are within the scope of the invention and claims.

## Claims

1. The use of an immunoadjuvant comprising a strain of Lactobacillus bacteria that has the ability to colonize the intestinal tract of the recipient, for the manufacture of an agent, for enhancing the immune response of a recipient to an oral vaccine.

2. The use of claim 1 wherein the Lactobacillus bacteria is a strain in which the bacteria have substantially the same properties exhibited by the Lactobacillus bacteria deposited in the American Type Culture Collection and given ATCC Accession No. 53103.

3. The use of claim 1 or claim 2 wherein the Lactobacillus bacteria is Lactobacillus Strain GG.

4. The use of any one of claims 1 to 3 wherein the agent comprises said immunoadjuvant together with a pharmaceutically acceptable carrier.

5. The use of claim 4 wherein the carrier is a fermented dairy product.

6. The use of claim 4 wherein the agent is in the form of culture-containing powder, capsule or tablet.

7. The use of any one of claims 1 to 6, wherein the immunoadjuvant comprises the bacteria in an amount sufficient to administer at least 10⁸ cfu.

8. A combined preparation containing at least two ingredients, one of which is an immunoadjuvant as defined in any one of claims 1 to 7, and the other is an oral vaccine for simultaneous, separate or sequential use of said two ingredients in enhancing the immune response to said oral vaccine, of a recipient of said vaccine.

9. The combined preparation as claimed in claim 8, for simultaneous use.

10. The combined preparation of claim 8 or claim 9 for simultaneous use followed by separate use of the immunoadjuvant alone.

11. The combined preparation of claim 10 wherein each administration of the immunoadjuvant contains a dosage of at least 10⁸ cfu of the bacteria.

## Patentansprüche

1. Verwendung eines Immunoadjuvans mit einem Stamm einer Lactobacillus-Bakterie, die die Fähigkeit aufweist, den Verdauungstrakt des Empfängers zu kolonisieren, um ein Agens herzustellen, um die Immunantwort eines Empfängers für ein orales Vakzin zu verstärken.

2. Verwendung nach Anspruch 1, wobei die Lactobacillus-Bakterie ein Stamm ist, bei dem die Bakterie im wesentlichen die gleichen Eigenschaften aufweist wie die Lactobacillus-Bakterie, die in der American Type Culture Collection hinterlegt ist und die ATCC-Eingangsnummer 53103 erhalten hat.

3. Verwendung nach Anspruch 1 oder Anspruch 2, bei der die Lactobacillus-Bakterie ein Lactobacillus Stamm GG ist.

4. Verwendung nach einem der Ansprüche 1 bis 3, bei der das Agens das besagte Immunoadjuvans zusammen mit einem pharmazeutisch akzeptablen Träger umfasst.

5. Verwendung nach Anspruch 4, bei dem der Träger ein fermentatives Milcherzeugnis ist.

6. Verwendung nach Anspruch 4, bei dem das Agens in der Gestalt eines die Kultur enthaltenden Pulvers, Kapsel oder Tablette vorliegt.

7. Verwendung nach einem der Ansprüche 1 bis 6, bei der das besagte Immunoadjuvans die Bakterie in einer Menge umfasst, um zumindest 10⁸ cfu zu verabreichen.

8. Ein kombiniertes Präparat mit zumindest zwei Ingredienzien, von denen eines ein Immunoadjuvans entsprechend einem der Ansprüche 1 bis 7 ist und von denen das andere ein orales Vakzin zur gleichzeitigen, getrennten oder aufeinander folgenden Verwendung der besagten zwei Ingredienzien zur Verbesserung der Immunantwort auf das besagte orale Vakzin für einen Empfänger des besagten Vakzins ist.

9. Kombiniertes Präparat nach Anspruch 8 zur gleichzeitigen Verwendung.

10. Kombiniertes Präparat nach Anspruch 8 oder Anspruch 9 zur gleichzeitigen Verwendung, gefolgt von einer getrennten Verwendung des Immunoadjuvans alleine.

11. Kombiniertes Präparat nach Anspruch 10, wobei jede Verabreichung des Immunoadjuvans eine Dosierung enthält, die mindestens 10⁸ cfu der Bakterie umfasst.

## Revendications

1. L'utilisation d'un immunoadjuvant comprenant une souche de bactéries Lactobacillus qui a l'aptitude à coloniser le tractus intestinal du receveur, pour la fabrication d'un agent en vue d'améliorer la réponse immunitaire d'un récepteur à un vaccin à administration par voie orale.

2. L'utilisation de la revendication 1 dans laquelle la bactérie Lactobacillus est une souche dans laquelle les bactéries ont sensiblement les mêmes propriétés que celles présentées par les bactéries Lactobacillus déposées à l'American Type Culture Collection et ayant reçu le numéro d'accession ATCC 53 103.

3. L'utilisation suivant la revendication 1 ou la revendication 2 dans laquelle la bactérie Lactobacillus est une souche de Lactobacillus GG.

4. L'utilisation suivant l'une quelconque des revendications 1 à 3 dans laquelle l'agent comprend l'immunoadjuvant ainsi qu'un véhicule acceptable pharmaceutiquement.

5. L'utilisation suivant la revendication 4 dans laquelle le véhicule est un produit laitier fermenté.

6. L'utilisation de la revendication 4 dans laquelle l'agent est sous la forme d'une poudre, d'une gélule ou d'un comprimé contenant une culture.

7. L'utilisation de l'une quelconque des revendications 1 à 6, dans laquelle l'immunoadjuvant comprend la bactérie en une quantité suffisante pour administrer au moins 10⁸ unités de formation de colonie.

8. Préparation combinée comprenant au moins deux ingrédients, l'un d'entre eux étant un immunoadjuvant tel que défini à l'une quelconque éventuellement des revendications 1 à 7, et l'autre, un vaccin oral pour l'utilisation simultanée, séparée ou séquentielle des deux ingrédients afin d'améliorer la réponse immunitaire au vaccin administré par voie orale d'un receveur du vaccin.

9. Préparation combinée suivant la revendication 8 destinée à une utilisation simultanée.

10. Préparation combinée suivant la revendication 8 ou la revendication 9 destinée à une utilisation simultanée, suivie d'une utilisation séparée de l'immunoadjuvant seul.

11. Préparation combinée suivant la revendication 10 dans laquelle chaque administration d'immunoadjuvant contient une dose d'au moins 10⁸ d'unités de formation de colonie de la bactérie.
